# DEMANDE DE BREVET EUROPEEN

(11) **EP 3 072 546 A1**
(43) Date de publication de la demande: **28.09.2016**
(21) Numéro de dépôt: 16156652.6
(22) Date de dépôt: 22.02.2016
(51) Int. Cl.: A61M 5/24, A61M 5/20

(54) **PORTE SERINGUE POUR DISPOSITIF D'INJECTION MOTORISÉ**

(30) Priorité: 25.03.2015 CH 4312015
(71) Demandeur: juvaplus SA, 1206 Geneve (CH)
(72) Inventeur: Legrand, Bernard-Pierre, 1231 Conches (CH); Konandreas, Stefanos, 1209 Petit-Saconnex (CH)
(74) Mandataire: Sammer, Thomas

(57) **Abrégé**

La présente invention concerne un porte seringue (1) pour dispositif d'injection motorisé (2), le porte seringue (1) étant apte à recevoir un récipient (3) destiné à contenir une substance fluide ou visqueuse, de préférence un récipient remplissable jetable ou une seringue, ainsi qu'apte à être monté sur ledit dispositif d'injection motorisé (2), de sorte à ce que le dispositif d'injection motorisé (2) permet d'expulser au moins une partie, voire la totalité de la substance fluide ou visqueuse contenue dans ledit récipient (3), le porte seringue (1) comprenant un premier moyen d'actionnement (1.1) permettant de commander une fonction prédéfinie dudit dispositif d'injection motorisé (2). Le porte seringue (1) se distingue du fait qu'il comprend un deuxième moyen d'actionnement (1.2) permettant de commander, de manière indépendante du premier moyen d'actionnement (1.1), ladite fonction prédéfinie du dispositif d'injection motorisé (2). La présente invention concerne également un dispositif d'injection motorisé (2) apte à recevoir un tel porte seringue (2) et comportant un nombre de boutons de commande positionnés sur la surface supérieure dudit dispositif d'injection motorisé (2) d'une manière spécifique.

## Description

### Champs de l'invention

La présente invention a pour objet un porte seringue pour dispositif d'injection motorisé, le porte seringue étant apte à recevoir un récipient destiné à contenir une substance fluide ou visqueuse, de préférence un récipient remplissable jetable ou une seringue, ainsi qu'apte à être monté sur ledit dispositif d'injection motorisé, de sorte à ce que le dispositif d'injection motorisé permet d'expulser au moins une partie, voire la totalité de la substance fluide ou visqueuse contenue dans ledit récipient, le porte seringue comprenant un premier moyen d'actionnement permettant de commander une fonction prédéfinie dudit dispositif d'injection motorisé. La présente invention concerne également un dispositif d'injection motorisé apte à recevoir un tel porte seringue.

### État de l'art antérieur

Dans ce contexte, il convient de noter que la demande de brevet européen EP 2 689 793 divulgue un dispositif d'injection équipé d'une source d'énergie, d'un système moteur alimenté par ladite source d'énergie et entraînant un premier piston propre au système moteur, et d'un porte seringue apte à être monté sur ledit système moteur et permettant de recevoir des seringues ou des récipients de différents types. Ainsi, une fois le système moteur du dispositif d'injection activé, ledit premier piston permet d'éjecter, en appuyant sur le deuxième piston propre à la seringue voire au récipient logé dans le porte seringue, de façon contrôlée et sans effort pour l'utilisateur un produit contenu dans la seringue, respectivement dans le récipient, c'est-à-dire de l'injecter dans le corps d'un patient.

Ce dispositif d'injection a été conçu pour faciliter le travail du praticien qui peut concentrer son attention et son effort à l'injection du produit et au patient au lieu de devoir consacrer une partie importante de son attention à la manutention d'une seringue de type conventionnelle ainsi que de devoir exercer, en fonction de la viscosité du produit à injecter, une force plus ou moins importante sur le piston de la seringue en y appuyant manuellement. Le dispositif permet simultanément d'utiliser la plupart des seringues conventionnelles, étant donné qu'il est équipé dudit porte seringue qui sert à monter les seringues sur le boîtier du système moteur du dispositif d'injection. Le dispositif ne peut pas seulement être utilisé en combinaison avec plusieurs types de seringues pré-remplies d'un produit à injecter, mais également avec des seringues voire d'autres types de récipients que le praticien doit remplir juste avant l'utilisation. En effet, certaines applications nécessitent la préparation du produit juste avant son utilisation, comme par exemple la toxine botulique qui est couramment utilisé dans la médecine esthétique.

Pour obtenir des performances optimales du dispositif d'injection, il est important que le praticien dispose d'une visibilité optimale sur le récipient contenant le produit à injecter, qu'il ait une prise en main optimale au niveau ergonomique, ainsi qu'il puisse activer le dispositif sans trop d'effort, étant donné que l'opération qu'il doit effectuer nécessite souvent une grande précision, notamment dans le domaine de la médecine esthétique. Si ledit dispositif d'injection selon le document EP 2 689 793 est déjà bien agencé de sorte à réduire l'effort necessaire du coté du praticien, étant donné qu'il dispose d'un système moteur permettant d'entraîner ledit premier piston et évitant donc au praticien de fournir lui-même la force nécessaire pour l'éjection du produit dudit récipient, la pratique a montré qu'il existent certains applications pour lesquelles le porte seringue mentionné ci-dessus ne permet pas de tirer tous les avantages de ce dispositif d'injection. En effet, d'une part, il existent des cas de figure où le praticien souhaiterait disposer d'un effort encore réduit par rapport à ce qui est offert par le porte seringue du système suscité. D'autre part, il existent également des cas de figure où l'ergonomie de ce porte seringue et la visibilité en découlant ne sont pas idéale.

Il est donc à constater que, à la connaissance du déposant, l'art antérieur ne comprend actuellement pas de moyen de support qui pourrait être utilisé de manière convenable en combinaison avec le dispositif d'injection suscité et qui permettrait au praticien de disposer d'un comfort encore amélioré en comparaison avec le porte seringue connu mentionné ci-dessus.

### Objectifs de l'invention

Le but de la présente invention est donc de remédier aux inconvénients susmentionnés et de mettre à disposition des praticiens utilisant des dispositifs d'injection motorisés mentionnés ci-dessus un porte seringue apte à être monté sur ce type de dispositifs d'injection et permettant de réduire encore l'effort nécessaire pour activer le dispositif, respectivement pour injecter ensuite le produit contenu dans le récipient logé dans le porte seringue. Un autre but de la présente invention est de réaliser ce porte seringue, c'est-à-dire le moyen de support d'un récipient destiné à être monté sur le boîtier d'un dispositif d'injection motorisé, tout en améliorant la visibilité sur le récipient contenant le produit à injecter ainsi que la prise en main du dispositif d'injection. Il est aussi un objectif de la présente invention de réaliser ce moyen de support de manière simple, à coûts modérés, ainsi que de sorte à ce qu'il soit fiable et flexible lors de son utilisation. Un autre objectif de la présente invention est de réaliser un dispositif d'injection qui est idéalement adapté à ce type de porte seringue.

### Solution selon l'invention

A cet effet, la présente invention propose un porte seringue, respectivement un dispositif d'injection motorisé du type susmentionné qui se distinguent par les caractéristiques énoncées à la revendication 1, respectivement à la revendication 7. En particulier, un porte seringue selon la présente invention se distingue du fait qu'il comporte un deuxième moyen d'actionnement permettant de commander, de manière indépendante du premier moyen d'actionnement, ladite fonction prédéfinie du dispositif d'injection motorisé, et le dispositif d'injection motorisé se distingue du fait qu'il comporte au moins un bouton d'activation positionné sur la surface supérieure dudit dispositif d'injection motorisé d'une manière spécifique.

Par ces mesures, le porte seringue permet une utilisation optimale d'un dispositif d'injection motorisé du type suscité car il réduit encore l'effort nécessaire de la part du praticien pour activer le dispositif, respectivement pour injecter ensuite le produit contenu dans le récipient logé dans le porte seringue, étant donné que même l'activation peut ainsi être effectuée avec un effort minimal en utilisant le deuxième moyen d'actionnement, la force nécessaire pour l'éjection du produit hors du récipient étant de toute façon fournie par le système moteur du dispositif. Par ailleurs, ce porte seringue améliore simultanément la prise en main du dispositif d'injection motorisé, étant donné que le praticien a le choix d'utiliser le premier - et/ou le deuxième moyen d'actionnement situés sur le porte seringue, ainsi que, pour la même raison, la visibilité sur le récipient contenant le produit à injecter. On obtient ainsi un moyen de support qui est adapté de manière optimale pour utilisation en combinaison avec un dispositif d'injection motorisé, ce porte seringue étant néanmoins simple de construction, peu couteux en fabrication, ainsi que robuste et fiable lors de l'utilisation.

D'autres caractéristiques, ainsi que les avantages correspondants, ressortiront des revendications dépendantes, ainsi que de la description exposant ci-après l'invention plus en détail.

### Description brève des dessins

Les dessins annexés représentent schématiquement et à titre d'exemple une forme d'exécution de l'invention.

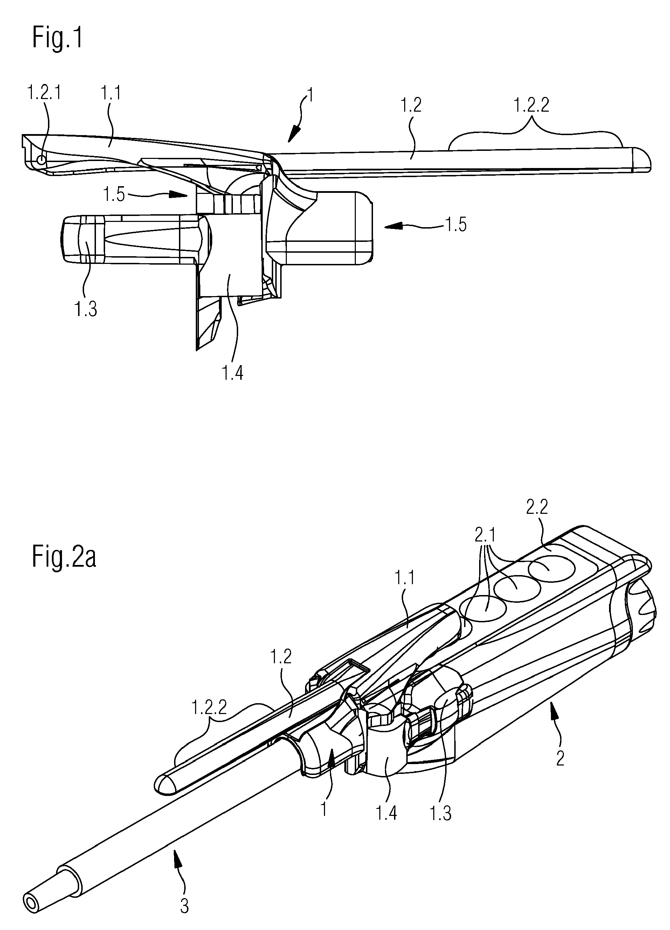

La figure 1 représente une vue schématique de côté d'un porte seringue selon la présente invention apte à être monté sur un dispositif d'injection motorisé.

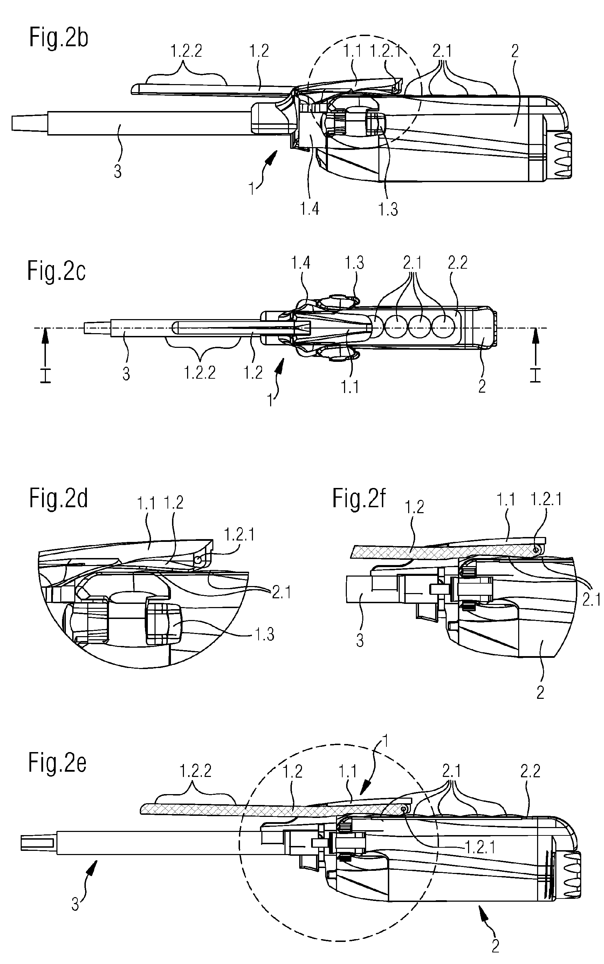

La figure 2a montre une vue schématique en perspective d'un porte seringue selon la présente invention dans un état monté sur un dispositif d'injection motorisé ainsi qu'en hébergeant un récipient contenant un produit destiné à être injecté dans le corps d'un patient; la figure 2b est une vue schématique de côté du porte seringue de la figure 2a y compris ledit dispositif d'injection motorisé ainsi que le récipient; la figure 2c est une vue schématique de dessus du porte seringue de la figure 2a y compris ledit dispositif d'injection motorisé ainsi que le récipient; la figure 2d est un agrandissement d'une partie de la figure 2b montrant plus en détail le porte seringue selon la présente invention; la figure 2e est une coupe longitunale, dont certaines parties sont illustrées en vue transparente pour simplifier la compréhension, à travers le porte seringue, le dispositif d'injection motorisé, ainsi que le récipient le long de la ligne I-I indiquée dans la figure 2c; la figure 2f est un agrandissement d'une partie de la figure 2e montrant plus en détail le porte seringue selon la présente invention.

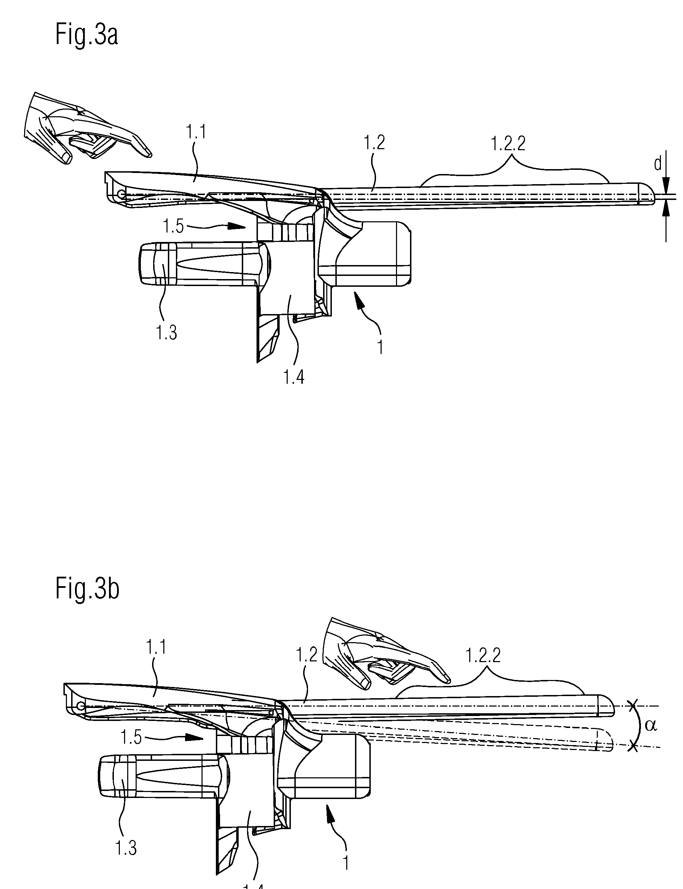

La figure 3a illustre, par une vue schématique de côté d'un porte seringue selon la présente invention, la commande d'un dispositif d'injection motorisé par l'intermédiaire de l'actionnement du premier moyen d'actionnement du porte seringue; la figure 3b illustre, par une vue schématique de côté et analogique à la figure 3a d'un porte seringue selon la présente invention, la commande d'un dispositif d'injection motorisé par l'intermédiaire de l'actionnement du deuxième moyen d'actionnement du porte seringue.

### Description détaillée de l'invention

L'invention sera maintenant décrite en détail en référence aux dessins annexés illustrant à titre d'exemple une forme d'exécution de l'invention.

La présente invention se rapporte à un porte seringue 1 pour dispositif d'injection motorisé 2, le porte seringue 1 étant apte à recevoir un récipient 3 destiné à contenir une substance fluide ou visqueuse, voire généralement un produit utilisé en medecine générale ou en médecine esthétique pour injection dans le corps d'un patient, ainsi qu'apte à être monté sur ledit dispositif d'injection motorisé 2. Le dispositif d'injection motorisé 2 permet d'expulser au moins une partie, voire la totalité du produit, respectivement de la substance fluide ou visqueuse contenu dans ledit récipient 3 en comprenant un système moteur alimenté par une source d'énergie et entraînant un premier piston propre au système moteur, ledit premier piston appuyant sur un deuxième piston propre au récipient 3, voire à la seringue logé dans le porte seringue 1. Un tel dispositif d'injection motorisé 2 ainsi qu'un récipient 3 correspondant, prenant souvent la forme d'une seringue conventionnelle ou, de préférence, d'un récipient remplissable jetable, sont décrits en détail dans le document EP 2 689 793 qui est intégré dans la présente description par référence, ces éléments n'étant par conséquent pas décrit plus en détail par la suite. Le porte seringue 1, tel que connu dans l'art antérieur et divulgué notamment de même dans le document EP 2 689 793, comprend un premier moyen d'actionnement 1.1 permettant de commander une fonction prédéfinie dudit dispositif d'injection motorisé 2.

Cette fonction prédéfinie consiste normalement en la mise en marche et l'arrêt du moteur du dispositif d'injection motorisé 2 et dans ce cas, suite à l'actionnement dudit premier moyen d'actionnement 1.1 du porte seringue 1, ce moyen appuie, lorsque le porte seringue 1 est monté sur le dispositif d'injection motorisé 2, sur un bouton de commande 2.1 correspondant dudit dispositif 2, ce bouton 2.1 commandant la mise en marche et l'arrêt du premier piston du dispositif d'injection motorisé 2. Ainsi, suite à un premier actionnement dudit premier moyen d'actionnement 1.1 du porte seringue 1, le moteur du dispositif 2 entraîne le premier piston propre au système moteur qui appuie à son tour sur le deuxième piston propre à la seringue, respectivement au récipient 3, logé dans le porte seringue 1. Le produit contenu dans le récipient, voire la seringue, est alors éjecté du récipient, respectivement injecté dans le corps d'un patient. Un deuxième actionnement dudit premier moyen d'actionnement 1.1 du porte seringue 1 permet ensuite d'arrêter le moteur du dispositif 2, respectivement d'arrêter le premier piston propre au système moteur ainsi que le deuxième piston propre à la seringue, de sorte à ce que l'injection du produit dans le corps du patient s'arrête également. De préférence, le bouton 2.1 commandant la mise en marche et l'arrêt du premier piston du dispositif d'injection motorisé 2 est agencé de sorte à ce qu'une pression sur ce bouton 2.1 provoque l'injection du produit pendant tout le temps qu'il est actionné et provoque l'arrêt de l'injection du produit lorsque la pression sur le bouton de commande 2.1 est relâchée. La fonction prédéfinie peut pourtant aussi consister en une autre fonction.

En effet, le porte seringue 1 pour dispositif d'injection motorisé 2 selon la présente invention comporte un corps principal 1.4 apte à recevoir un récipient 3, tel qu'un récipient remplissable jetable ou une seringue conventionnelle, en comprenant une ouverture 1.5 traversant axialement le porte seringue 1 dans laquelle le récipient 3 peut être inséré, ce qui est illustré schématiquement à la figure 1. De plus, le porte seringue 1 est évidemment apte à être monté sur le dispositif d'injection motorisé 2, par exemple à l'aide de languettes de fixation latérales 1.3 ou toute autre moyen de fixation similaire. Étant donné que le premier piston propre au système moteur du dispositif d'injection 2 peut traverser ladite ouverture 1.5 du porte seringue 1 afin de pénétrer dans l'intérieur du récipient 3, afin d'appuyer sur le deuxième piston du récipient 3, le dispositif d'injection motorisé 2 permet d'expulser au moins une partie, voire la totalité de la substance fluide ou visqueuse contenue dans ledit récipient 3, une fois que le porte seringue 1 avec un récipient 3 contenant un produit a été monté sur le dispositif 2. Les détails et alternatives techniques concernant la fixation du récipient 3 dans le porte seringue 1 ainsi que les moyens de fixation et le montage du porte seringue 1 sur le dispositif d'injection motorisé 2 ne seront pas discutés par la suite, étant donné que, d'une part, ces informations se trouvent dans le document EP 2 689 793 et que, d'autre part, cela n'a pas d'influence majeure sur la présente invention.

Cette invention réside principalement dans le fait qu'un tel porte seringue 1 comporte un deuxième moyen d'actionnement 1.2 permettant également de commander, de manière indépendante du premier moyen d'actionnement 1.1, ladite fonction prédéfinie du dispositif d'injection motorisé 2. Tel que cela est illustré schématiquement et à titre d'exemple à la vue de côté de la figure 1, ce deuxième moyen d'actionnement 1.2 du porte seringue 1 est réalisé, de préférence, par un levier d'actionnement 1.2 articulé sur le premier moyen d'actionnement 1.1. L'axe d'articulation 1.2.1 peut être réalisé par un axe fixé au premier moyen d'actionnement 1.1 et logé dans un trou traversant du levier d'actionnement 1.2 ou, inversement, par un axe fixé au levier d'actionnement 1.2 dont les extrémités sont logées dans des trous correspondants formés dans la partie inférieure de l'extrémité libre dudit premier moyen d'actionnement 1.1 du porte seringue 1, cette l'extrémité libre étant orientée vers l'extrémité proximale du dispositif d'injection motorisé 2. L'axe d'articulation 1.2.1 peut également être réalisé par des saillies cylindriques ou coniques formées d'une pièce avec le levier d'actionnement 1.2, ces saillies étant logées dans des trous correspondants formés dans ladite extrémité libre dudit premier moyen d'actionnement 1.1 du porte seringue 1 ou, inversement, par des saillies formées d'une pièce avec le premier moyen d'actionnement 1.1, ces saillies étant logées dans des trous correspondants formés dans le levier d'actionnement 1.2.

L'extrémité libre du levier formant le deuxième moyen d'actionnement 1.2 du porte seringue 1 est orientée vers l'extrémité distale du récipient 3 monté dans le porte seringue 1, de sorte à ce que ce levier d'actionnement 1.2 s'étend sensiblement parallèlement au récipient 3, en présentant une zone d'activation 1.2.2 le long de laquelle l'utilisateur du dispositif peut appuyer à son gré, notamment en fonction de la taille de sa main ainsi que de la manière à laquelle il souhaite tenir le dispositif d'injection motorisé 2 y compris le porte seringue 1 et la seringue 3 installé dans ce dernier. L'extrémité proximale du levier formant le deuxième moyen d'actionnement 1.2 du porte seringue 1 articulé au premier moyen d'actionnement 1.1 est monté en dessous de ce dernier, tel qu'illustré également aux figures 2a et 2b. Il ressort notamment de la figure 2d, qui montre de manière aggrandi un détail de la figure 2b, qu'en état monté sur le dispositif d'injection motorisé 2, une partie du levier formant le deuxième moyen d'actionnement 1.2 du porte seringue 1 proche de l'axe d'articulation 1.2.1 est superposée au bouton de commande 2.1 du dispositif d'injection motorisé 2 permettant d'activer et d'arrêter ladite fonction prédéfinie du dispositif 2. De son tour, le premier moyen d'actionnement 1.1 est superposé à l'extrémité proximale du levier formant le deuxième moyen d'actionnement 1.2 du porte seringue 1 et couvrant ledit bouton de commande 2.1 du dispositif d'injection motorisé 2. Ainsi, ce bouton de commande 2.1 du dispositif d'injection motorisé 2 permettant d'activer et d'arrêter ladite fonction prédéfinie du dispositif 2 peut être actionné soit directement par le deuxième moyen d'actionnement 1.2 du porte seringue 1, en appuyant à un endroit donné situé le long de sa zone d'activation 1.2.2, soit indirectement en appuyant sur le premier moyen d'actionnement 1.1 du porte seringue 1 qui déplace le deuxième moyen d'actionnement 1.2 situé sous sa surface inférieure et articulé à son extrémité libre. Le deuxième moyen d'actionnement 1.2 du porte seringue 1 est, de préférence, maintenu dans sa position de repos, dans laquelle il n'appuie pas sur le bouton de commande 2.1 du dispositif d'injection motorisé 2 permettant d'activer et d'arrêter ladite fonction prédéfinie du dispositif 2, par un ressort de précontrainte situé entre le premier moyen d'actionnement 1.1 et le deuxième moyen d'actionnement 1.2, par déformation élastique des pieds latéraux liant le premier moyen d'actionnement 1.1 au corps principal 1.4, entre lesquels est logé le deuxième moyen d'actionnement 1.2, ou par toute autre moyen adéquat.

De préférence, le levier d'actionnement 1.2 est formé par une tige d'une longueur totale de 40 mm à 90 mm environ, de préférence de 50 mm à 70 mm, de sorte à ce que l'extrémité libre de la tige dépassant le premier moyen d'actionnement 1.1 devrait couvrir moins de la moitié de la longueur du récipient 3, respectivement de la seringue. Son extrémité orientée vers l'extrémité distale du récipient 3 sera donc situé environ dans le deuxième quart ou dans le deuxième tiers de la longueur totale du dispositif d'injection motorisé 2 y compris le porte seringue 1 et la seringue 3. Par conséquent, la force d'activation de ladite fonction prédéfinie requise par le deuxième moyen d'actionnement 1.2 du porte seringue 1 est inférieure à la force d'activation requise par le premier moyen d'actionnement 1.1 du porte seringue 1, car l'effet levier favorable procuré par la multiplication de la force d'appui en fonction de l'endroit où exactement appuie l'utilisateur le long de la zone d'activation 1.2.2 du deuxième moyen d'actionnement 1.2 permet une activation dudit bouton de commande 2.1 nécessitant quasiment pas d'effort de la part de l'utilisateur. De plus, l'utiisateur peut, en choisissant l'endroit où exactement il appuie le long de la zone d'activation 1.2.2 du deuxième moyen d'actionnement 1.2 du porte seringue 1, choisir graduellement l'effort requis pour activer la fonction prédéfinie, ce qui ressort plus particulièrement des vues en coupe des figures 2e et 2f.

En ce qui concerne le premier moyen d'actionnement 1.1, il est d'abord à noter qu'il est agencé sur le corps principal 1.4 du porte seringue 1 de sorte à être orienté vers l'extrémité du porte seringue 1 destinée à être montée sur ledit dispositif d'injection motorisé 2. De préférence, le premier moyen d'actionnement 1.1 forme une surface de protection couvrant, lorsque le porte seringue 1 est monté sur le dispositif d'injection motorisé 2, au moins une partie des boutons de commande 2.1 positionnés sur la surface supérieure 2.2 dudit dispositif d'injection motorisé 2, afin d'éviter toute actionnement involontaire des boutons de commande 2.1 placé en dessous de cette surface de protection. Par ailleurs, le premier moyen d'actionnement 1.1 peut avantageusement être formé d'une pièce avec le corps principal 1.4 du porte seringue 1, avec lequel il est notamment lié par des pieds latéraux tel qu'illustré par exemple aux figures 2a et 2c. Dans ce cas, le premier moyen d'actionnement 1.1 est actionnable par déformation élastique aussi bien de son extrémité proximale que des desdits pieds latéraux, de sorte à pouvoir appuyer, par l'intermédiaire du deuxième moyen d'actionnement 1.2, sur un des boutons de commande 2.1 positionnés sur la surface supérieure 2.2 dudit dispositif d'injection motorisé 2 afin de commander ladite fonction prédéfinie dudit dispositif d'injection motorisé 2.

Au vue de la description figurant ci-dessus de la structure d'un porte seringue selon la présente invention, le fonctionnement de ce type de moyen de support en combinaison avec un dispositif d'injection motorisé 2 est aisément compréhensible. En effet, tel qu'illustré à la figure 3a par une vue schématique de côté d'un porte seringue selon la présente invention, il est possible de commander un dispositif d'injection motorisé 2 du type mentionné dans l'introduction par l'intermédiaire de l'actionnement du premier moyen d'actionnement 1.1 du porte seringue 1, simplement en appuyant sur ce dernier. Alternativement, tel qu'illustré à la figure 3b par une vue schématique de côté et analogique à la figure 3a, il est également possible de commander un tel dispositif d'injection motorisé 2 par l'intermédiaire de l'actionnement du deuxième moyen d'actionnement 1.2 du porte seringue 1, également simplement en appuyant sur la zone d'activation 1.2.2 de dernier, cela nécessitant à la fois une force d'activation inférieure ainsi que plus dosable. Il ressort d'ailleurs des figures 3a et 3b que, en utilisant le premier moyen d'actionnement 1.1 du porte seringue 1, le levier de commande 1.2 est déplacé sensiblement parallèlement par rapport à sa position de repos, à savoir d'une distance d, jusqu'à ce qu'il appuie sur le bouton de commande 2.1 positionné sur la surface supérieure 2.2 dudit dispositif d'injection motorisé 2 et commandant ladite fonction prédéfinie, tandis que, en utilisant le deuxième moyen d'actionnement 1.1 du porte seringue 1, le levier de commande 1.2 est déplacé angulairement d'un angle α autour de l'axe d'articulation 1.2.1, de même jusqu'à ce qu'il appuie sur le bouton de commande 2.1 du dispositif d'injection motorisé 2.

Il reste à noter que la présente invention concerne également un dispositif d'injection motorisé 2 apte à recevoir un tel porte seringue 1, ce dispositif 2 comportant un nombre prédéfini de boutons de commande 2.1 positionnés sur sa surface supérieure et contrôlant les différentes fonctions du dispositif d'injection motorisé 2. En particulier, dans un dispositif d'injection motorisé 2 selon l'invention, au moins une partie des boutons de commande 2.1 contrôlant des fonctions pouvant toucher à la sécurité du dispositif d'injection 2 sont positionnés sur la surface supérieure 2.2 du dispositif de manière à être couvert, lorsque le porte seringue 1 est monté sur le dispositif d'injection motorisé 2, par la surface de protection formée par le premier moyen d'actionnement 1.1 du porte seringue 1. En particulier, les boutons de commande 2.1 contrôlant des fonctions pouvant toucher à la sécurité du dispositif et positionnés sur la surface supérieure 2.2 du dispositif de manière à être couvert, lorsque le porte seringue 1 est monté sur le dispositif d'injection motorisé 2, par la surface de protection formée par ledit premier moyen d'actionnement 1.1 du porte seringue 1 peuvent consister en le bouton de mise en marche et d'arrêt du dispositif d'injection motorisé 2, le bouton de mise en marche et d'arrêt du premier piston du dispositif d'injection motorisé 2, le bouton de remise à la position zéro du premier piston du dispositif d'injection motorisé 2, et/ou le bouton de reset de certains paramètres du dispositif d'injection motorisé 2. Dans un tel dispositif d'injection motorisé 2, ces boutons de commande 2.1 sont donc agencés sur la surface supérieure 2.2 du dispositif 2 dans un ordre choisi et adapté en fonction de l'application concrète pour laquelle le dispositif est prévu. Par exemple, un agencement favorable des boutons de commande 2.1 contrôlant les différents fonctions du dispositif d'injection motorisé 2 peut consister en plaçant, dans l'ordre et en commençant sur l'extrémité distale du dispositif 2, d'abord le bouton de remise à la position zéro du premier piston du dispositif d'injection motorisé 2, ensuite le bouton de mise en marche et d'arrêt du premier piston du dispositif d'injection motorisé 2, suivi par des boutons contrôlant la vitesse d'injection du produit, c'est-à-dire la vitesse de déplacement du premier piston du dispositif d'injection motorisé 2 et/ou encore d'autres boutons de commande, pour terminer avec le bouton de mise en marche et d'arrêt du dispositif d'injection motorisé 2. Ce dernier et/ou le bouton de reset de certains paramètres du dispositif d'injection motorisé 2 peuvent aussi être placé en première ou en deuxième position comptée à partir de l'extrémité distale du dispositif 2. De sorte, en fonction de la longueur de la surface de protection formée par le premier moyen d'actionnement 1.1 du porte seringue 1, le ou les premiers boutons de commande 2.1 sur l'extrémité distale du dispositif 2 ne sont actionnables que lorsque le porte seringue 1 est enlevé du dispositif d'injection motorisé 2, évitant ainsi tout actionnement involontaire de ces boutons pendant que le porte seringue 1 est monté sur le dispositif d'injection motorisé 2 et, notamment, lors de la marche du dispositif 2, tandis que le bouton de commande 2.1 commandant la fonction prédéfinie et situé sous la zone de commande du premier moyen d'actionnement 1.1 peut être commandé, tel qu'expliqué ci-dessus, soit par le premier moyen d'actionnement 1.1 soit par le deuxième moyen d'actionnement 1.2 du porte seringue 1.

Au vu des explications figurant ci-dessus, il est évident qu'un porte seringue selon l'invention permet une utilisation optimale d'un dispositif d'injection motorisé du type mentionné dans l'introduction tout en restant compatible avec tout type de récipient ou de seringue. En particulier, un tel un porte seringue réduit encore l'effort nécessaire de la part de l'utilisateur pour activer le dispositif d'injection, respectivement pour injecter ensuite le produit contenu dans le récipient logé dans le porte seringue, sachant que l'activation de la fonction souhaitée peut ainsi être effectuée avec un effort minimal en utilisant le deuxième moyen d'actionnement et que la force nécessaire pour l'injection du produit dans le corps du patient est de toute façon fournie par le système moteur du dispositif d'injection motorisé. De même, l'utilisateur peut de ce fait doser la quantité de produit injectée au patient plus finement et est en mesure de tenir le dispositif d'injection motorisé à la manière d'un stylo plume. De plus, il permet à l'utilisateur de choisir la manière à laquelle il voudrait commander le dispositif d'injection motorisé, soit avec le premier - ou avec le deuxième moyen d'actionnement, offrant ainsi plus de flexibilité au praticiens. Ce système d'activation permet alors à la fois une activation très douce grâce à l'effet levier favorable et une activation sur la partie centrale du système entier consistant en trois parties séparées montées l'une sur l'autre, à savoir en un récipient logé dans le porte seringue qui est monté sur le boîtier du dispositif d'injection motorisé. En même temps, cela améliore la maniabilité du dispositif d'injection motorisé, car le choix d'utiliser le premier - et/ou le deuxième moyen d'actionnement situés sur le porte seringue permet au praticien de choisir également la prise en main ergonomique qui lui convient au mieux. Par ailleurs, cela améliore également la visibilité sur le récipient contenant le produit à injecter, car il n'y a plus de boîtier à effet ergonomique défavorable mais une réduction au minimum de l'encombrement du porte seringue, permettant donc une meilleure visibilité pour lecture directe sur la seringue, sur la quantité de produit qu'elle contient encore, et, surtout, sur le patient. Le porte seringue est donc adapté de manière optimale pour utilisation en combinaison avec un tel dispositif d'injection motorisé. De plus, le porte seringue est de construction simple et robuste, peu couteux en fabrication, et fiable lors de son utilisation.

## Revendications

1. Porte seringue (1) pour dispositif d'injection motorisé (2), le porte seringue (1) étant apte à recevoir un récipient (3) destiné à contenir une substance fluide ou visqueuse, de préférence un récipient remplissable jetable ou une seringue, ainsi qu'apte à être monté sur ledit dispositif d'injection motorisé (2), de sorte à ce que le dispositif d'injection motorisé (2) permet d'expulser au moins une partie, voire la totalité de la substance fluide ou visqueuse contenue dans ledit récipient (3), le porte seringue (1) comprenant un premier moyen d'actionnement (1.1) permettant de commander une fonction prédéfinie dudit dispositif d'injection motorisé (2), **caractérisé par le fait que** le porte seringue (1) comprend un deuxième moyen d'actionnement (1.2) permettant de commander, de manière indépendante du premier moyen d'actionnement (1.1), ladite fonction prédéfinie du dispositif d'injection motorisé (2).

2. Porte seringue (1) selon la revendication précédente, **caractérisé par le fait que** ledit deuxième moyen d'actionnement (1.2) du porte seringue (1) est réalisé par un levier articulé sur ledit premier moyen d'actionnement (1.1).

3. Porte seringue (1) selon la revendication précédente, **caractérisé par le fait que** l'extémité libre dudit levier formant le deuxième moyen d'actionnement (1.2) du porte seringue (1) est orientée vers l'extrémité distale du récipient (3) monté dans le porte seringue (1).

4. Porte seringue (1) selon selon l'une des revendications précédentes, **caractérisé par le fait que** la force d'activation de ladite fonction prédéfinie requise par le deuxième moyen d'actionnement (1.2) du porte seringue (1) est inférieure à la force d'activation requise par le premier moyen d'actionnement (1.1) du porte seringue (1).

5. Porte seringue (1) selon l'une des revendications précédentes, **caractérisé par le fait que** ledit premier moyen d'actionnement (1.1) est orienté vers l'extrémité du porte seringue (1) destinée à être montée sur ledit dispositif d'injection motorisé (2) et forme une surface de protection couvrant, lorsque le porte seringue (1) est monté sur le dispositif d'injection motorisé (2), au moins une partie des boutons de commande (2.1) positionnés sur la surface supérieure (2.2) dudit dispositif d'injection motorisé (2).

6. Porte seringue (1) selon l'une des revendications précédentes, **caractérisé par le fait que** ledit premier moyen d'actionnement (1.1) est formé d'une pièce avec le corps principal (1.4) du porte seringue (1) et actionnable par déformation, de sorte à pouvoir appuyer sur un des boutons de commande (2.1) positionnés sur la surface supérieure (2.2) dudit dispositif d'injection motorisé (2) afin de commander ladite fonction prédéfinie dudit dispositif d'injection motorisé (2).

7. Dispositif d'injection motorisé (2) apte à recevoir un porte seringue (1) selon l'une des revendications précédentes et comportant un nombre prédéfini de boutons de commande (2.1) positionnés sur la surface supérieure du dispositif et contrôlant les fonctions du dispositif d'injection motorisé (2), **caractérisé par le fait qu'**au moins une partie des boutons de commande (2.1) contrôlant des fonctions pouvant toucher à la sécurité du dispositif d'injection (2) sont positionnés sur la surface supérieure (2.2) du dispositif de manière à être couvert, lorsque le porte seringue (1) est monté sur le dispositif d'injection motorisé (2), par la surface de protection formée par ledit premier moyen d'actionnement (1.1) du porte seringue (1).

8. Dispositif d'injection motorisé (2) selon la revendication précédente, **caractérisé par le fait que** les boutons de commande (2.1) contrôlant des fonctions pouvant toucher à la sécurité du dispositif et positionnés sur la surface supérieure (2.2) du dispositif de manière à être couvert, lorsque le porte seringue (1) est monté sur le dispositif d'injection motorisé (2), par la surface de protection formée par ledit premier moyen d'actionnement (1.1) du porte seringue (1) sont choisis parmi le groupe comprenant le bouton de mise en marche et d'arrêt du dispositif d'injection motorisé (2), le bouton de mise en marche et d'arrêt du premier piston du dispositif d'injection motorisé (2), le bouton de remise à la position zéro du premier piston du dispositif d'injection motorisé (2), le bouton de reset du dispositif d'injection motorisé (2).
